Europäisches Patentamt

European Patent Office

Office européen des brevets

(19)

(11) Publication number: **0 033 058**
**A1**

# (12) EUROPEAN PATENT APPLICATION

(21) Application number: **80830005.7**

(22) Date of filing: **29.01.80**

(51) Int. Cl.³: **G 05 D 22/02**
**B 02 B 1/04, G 01 N 33/10**

(43) Date of publication of application:
**05.08.81 Bulletin 81/31**

(84) Designated Contracting States:
**CH DE FR GB**

(71) Applicant: **SANGATI S.p.A.**
**Via Tiziano Aspetti, 232**
**I-35100 Padova(IT)**

(72) Inventor: **Bolognesi, Novello**
**Via Italo Piccagli, 6**
**Grassina (Firenze)(IT)**

(74) Representative: **Rapisardi, Raffaello**
**L.go Vo Alpini, 15**
**I-20145 Milano(IT)**

(54) **Automatic apparatus for moistening the wheat.**

(57) A suitable member (1) is set into the feeding duct (2) of the wheat to the humidifier, to continuously take the percentage of humidity in the wheat to be moistened. The results thus obtained are first elaborated and then used as parameters of comparison to continuously actuate a dosing device of the moistening water.

Dosing continuously the correct quantity of bathing water to the wheat to be milled, it is possible to reach the useful percentage of relative humidity to obtain the perfect milling of the wheat.

./...

EP 0 033 058 A1

Fig. 1

- 1 -

## AUTOMATIC APPARATUS FOR MOISTENING THE WHEAT.

The present invention relates to an automatic apparatus for moistening the wheat.

It is known in the art that the wheat to be milled contains from 12% to 15% in weight of moisture; said percentage depends on the coop and on the locality of growing.

It is also known that to obtain the perfect milling of the wheat, this latter must contain the optimum value of 17% in weight of humidity.

The machines of the prior art provide a manual moisten operation of the wheat, and consequently the obtained product doesn't contain the correct quantity of water.

It is an object of the present invention to provide an automatic apparatus to moisten the wheat through an automatic dosage of water to reach the useful value of 17% in weight of humidity in the wheat to be milled, the apparatus according to the present invention carrying out instantaneous measurements on the product to be moistened.

Such purpose is obtained, according to the invention, by means of an electronic device comprising a sensitive element set into the feeding duct of the wheat to the humidifier, to continuously take the percentage of humidity in the wheat to be moistened.

The results obtained from said measurements operations are first elaborated and then used as parameters of comparision to continuously actuate a dosing device of moistening water; the correct percentage of water is added to that one contained in the wheat to reach the suitable humidity in the product.

The wheat so moistened is let at rest for 18-20 hours, thus obtaining the suitable percentage of humidity for a good milling of the wheat.

The invention will be now described, by way of example, with reference to the accompanying drawings in which:

- fig. 1 is a front view, partially in section, of the humidity detector unit of the apparatus according to the invention;
- fig. 2 is a view, partially in section, of said detector;
- fig. 3 is a front view, partially in section, of the dosage unit of the apparatus of the invention;
- fig. 4 is a side view, partially in section, of said dosage unit;
- fig. 5 shows the circuit diagram of the control unit for the apparatus of the invention; and
- fig. 6 shows the feeder of said control unit.

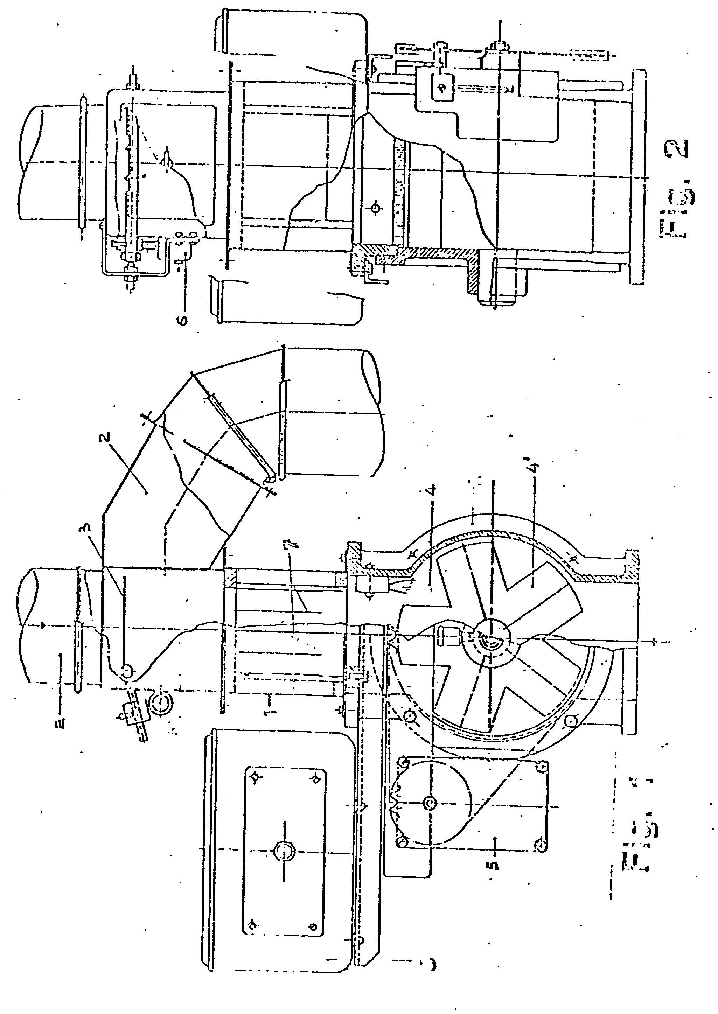

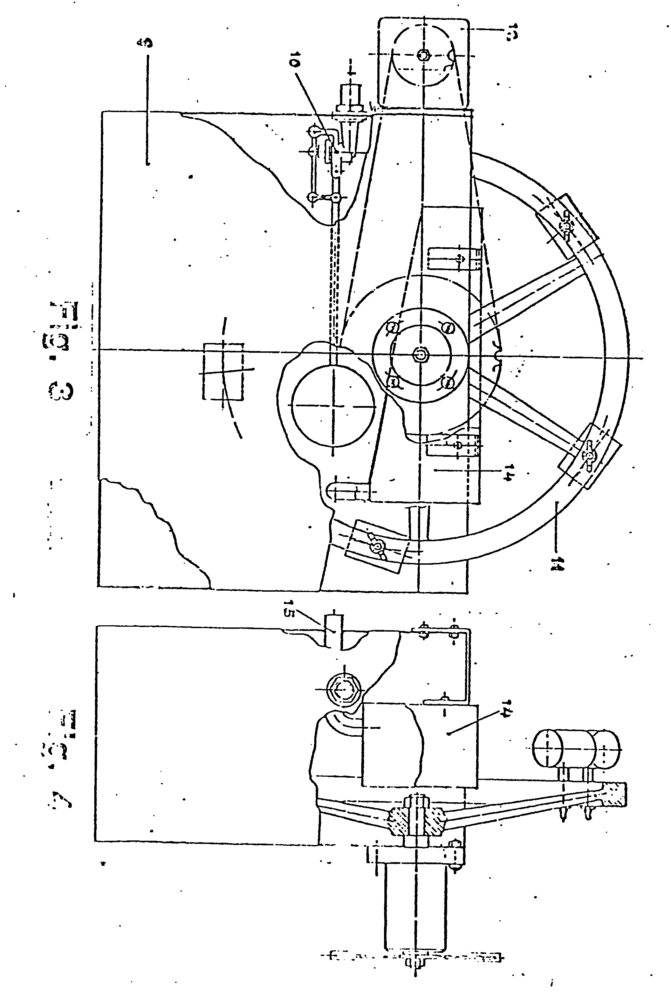

With reference to the cited drawings, an automatic apparatus to moisten the wheat according to the present invention substantially comprises:

a) a detector unit for measuring the instantaneous humidity percentage of the wheat to be treated. Said detector unit is formed by a cell 1 opened at the upper and lower sides, said cell being upwardly connected to a feeder duct 2 through a throttle valve 3 provided with a counterweight; the lower side of the cell 1 is connected to a rotary valve having spaces 4 alternated with solid projections 4'; said rotary valve rotates slowly with uniform speed, and it is operated by an electric motor 5, this latter being actuated by said valve 3 through a switch 6 which operates the electronic control unit. The cell 1 is formed by a crystal envelope and by five upright metallic plates 7 connected by turns, said plate 7 representing the sensitive element of an electric control unit located within a case 8. A little quantity of wheat passes continuously with intermittent motion thtough the cell 1 and stops within said cell for a little time; the remaining part of the wheat to be treated proceeds into the duct 2. The intermittent motion of the little quantity of wheat in the cell 1 is due to the lower rotary valve: when under the cell 1 is set one of its spaces 4, the volume of which being equal to that of the cell 1, the wheat leaves the cell 1 itself; on the contrary the quantity of wheat contained within the cell 1 stops into this latter when the rotary valve is set with one of its projection 4' under the discharge section of the cell 1; the wheat portion passing through the cell 1 into the spaces 4 of the rotary valve, leaves this latter and joines the remaining part of the wheat to be treated.

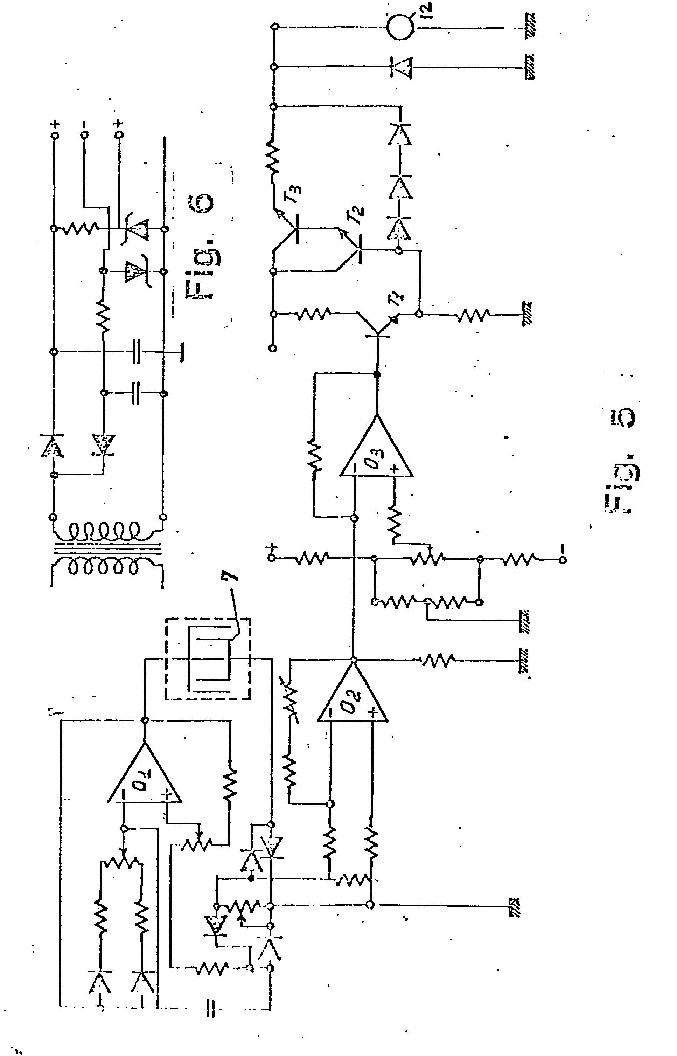

b) an electronic control unit formed by a circuit which provides the test voltage at the plates 7 of the sensitive element; said circuit substantially comprises an amplifier 01, a tension amplifying unit formed by two operating phases 02 and 03, a terminal regulator formed by a first separating transistor T1 and one couple of transistors T2 assembled in the Darlington system. Said circuit diagram is completed by the resistor groups, the condensers and the diodes assembled according to the diagram of fig. 5, which acts to polarize, stabilize, compensate, protect and couple the cited main components.

In operation a portion of the wheat to be treated is drawn by gravity from the duct 2 to fill the cell 1 lowering the valve 3 and causing the switch 6 closes and actuates the unit control: the electric motor 5 starts and operates the rotary valve 4 which controls the passage and the permanence of the wheat within the cell 1. The vertical plates 7 of the cell 1 are connected between them in groups of two, three of them, i.e. the first is connected to the third and the fifth, and the second is connected to the fourth; said plates are maintained at the alterna te voltage of the unit control.

Due to the presence of the wheat, the sensor element acts as an electric condenser, the plates of which being constituited by the plates 7, and the wheat representing the interposed dielectric the properties of which strictly depends on the humidity of the wheat itself.

Indeed the sensitive element act as a condenser with variable impedance, the value of the low electric current flowing into it depending proportionally on the percentage of humidity in the wheat.

Thus after a comparision value of the intensity of the electric current has been set (e.g. the intensity of current corresponding to the optimum value of 17% of humidity), any positive or negative variation of current from this value represents, by means of the electronic control unit, the regulation signal which operates the dosage unit.

Said regulation is realized controlling the revolving speed of the motor 22 which starts in rotation the wheel 11: in fact any modification of the revolving speed of said wheel, causes a variation on the quantity of water discharged into the container 14 and then distributed on the whole mass of wheat through the discharge duct 15.

More exactly the signal formed by the microamperometric current within the plates 7 of the sensitive element is supplied by the amplifier 01, rectified by the first operating phase 02, amplified and then compared in the second operating phase 03 with a signal of reference, said phase 03 acting as an amplifier of the difference of current.

The signal resulting from the comparision phase operates, through the transistor T1, the Darlington phase formed by the transistors T2 and T3, said Darlington phase providing the feeding voltage of the motor 12.

Any variation on the current intensity in the sensitive member

due to the corresponding variations of the humidity percentage in the wheat , provides a similar modification in the feeding voltage of the motor 12 by means of the circuit phases aforedescribed. Said voltage modification in the motor 12 provides a corresponding increasing or lowering of its speed, thus respectively increasing or lowering the quantity of water furnished from the cups of the wheel 11.

The herein described embodiments are illustrative, and it will be understood that further and equivalent modifications may be made without departing from the spirit and scope of the appended claims.

## Claims

1. An automatic apparatus for moistening the wheat, in which a control and data elaboration system is provided to continuously check the humidity value of a little portion of the product to be treated through a comparision step made between the value of the current flowing within e sensitive element and the value of a current of comparision, and to subsequently continuously regulate, by means of a capacity phase, the feeding voltage of a motor in d.c. provided to operate the dosing unit of the bathing water with an inverse proportion to the checked percentage of humidity.

2. An automatic apparatus as claimed in claim 1, wherein a detector unit is provided for measuring the instantaneous humidity of the wheat, said unit comprising a cell with vertical metallic plates acting as a sensitive element of an electronic control unit, said cell being filled with a corresponding quantity of wheat which is intermittently substituted, in a continuous cycle, by means of a rotary valve which rotates slowly and constantly by means of an electric motor, said valve further determining the time of permanence of said wheat portion within said cell by means of solid projections alternated with spaces, the volume of these latter being equal to that of said cell, the cited automatic apparatus further comprising a throttle valve located on the upper side of the cell, said throttle valve being operated by the portion of wheat to be tested, and being further connected to a switch operating the electronic control unit and the electric motor which actuated the cited rotary valve.

3. An automatic apparatus as claimed in claims 1 or 2, in which a unit for dosing the water is provided, said unit comprising a container fitted with a ball-cock, a wheel with fitted cups operated by an electric motor with controlled voltage, and a con-

tainer of the water collected by said wheel which is to be distributed in the wheat to be treated, the operation of said unit for dosing water being controlled by the control unit.

4. An apparatus as claimed in one or more of the preceding claims, in which an electronic control unit is provided first to elaborate the results of the measurements of the relative humidity in the wheat portion which passes through the cited cell, and then to regulate the revolving speed of the motor actuating the water dosing unit in inversely proportional manner compared with the water percentage measured on the portion of wheat which passes through the cell.

5. An apparatus as claimed in the precedings claims, wherein said control unit comprises the electronic circuit diagram shown in figures 5 and 6 of the annexed sheets of drawings, the fundamental components of which being an amplifier (01) which generates alternative signals, a second signal amplifier (02) and an operating phase (03) in cascade acting as an amplifier of difference, a separating transistor (T1) and a couple of transistors (T2) and (T3) assembled in the Darlington system acting as a tension regulating unit.

Fig. 2

Fig. 1

Fig. 3

Fig. 4

Fig. 6

Fig. 5

3|3

0033058

0033058

European Patent Office

**EUROPEAN SEARCH REPORT**

Application number

EP 80 83 0005

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| | US - A - 3 144 029 (C.F. STRAND-BERG) <br> * Whole patent * <br><br> -- | 1,4 | G 05 D 22/02 <br> B 02 B  1/04 <br> G 01 N 33/10 |
| | FR - A - 2 345 720 (FRANCE-LUZERNE) <br> * Page 3, line 4 - page 6, line 22; figures 1-4 * <br><br> -- | 1,2,5 | |
| | US - A - 3 841 610 (K. HANZAWA et al.) <br> * Column 1, lines 43-62; column 3, lines 51-63; figure 2 * <br><br> -- | 1,4,5 | TECHNICAL FIELDS SEARCHED (Int.Cl. 3) |
| | FR - A - 1 492 281 (PELLAM) <br> * Page 1, right-hand column, lines 1-20; page 3, left-hand column, lines 13-20; figure 5 * <br><br> -- | 2 | B 02 B  1/04 <br> G 05 D 22/02 <br> G 01 N 33/10 <br> 27/04 <br> 1/20 <br> F 04 B 19/10 |
| | GB - A - 14 004 AD 1912 (H. DEUTSCH) <br> * Page 1, lines 5-9; page 2, lines 19-34; page 3, lines 7-21; figures 1,2,7 * <br><br> -- | 2 | |
| | FR - A - 1 303 316 (CHEMIEBAU DR. A. ZIEREN) <br> * Whole patent * <br><br> -- | 3 | CATEGORY OF CITED DOCUMENTS <br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | GB - A - 5293 AD 1910 (J. ROWELL et al.) <br> * Page 1, lines 27-37; figures 1,2 * <br> ---- | 3 | &: member of the same patent family, corresponding document |

The present search report has been drawn up for all claims

| Place of search | Date of completion of the search | Examiner |
|---|---|---|
| The Hague | 22-09-1980 | POINT |

EPO Form 1503.1  06.78